# EUROPÄISCHE PATENTANMELDUNG

(11) **EP 4 841 549 A1**
(43) Veröffentlichungstag der Anmeldung: **18.11.2026**
(21) Anmeldenummer: 25176286.0
(22) Anmeldetag: 14.05.2025
(51) Int. Cl.: G06Q 50/08, G06K 19/07, B28B 23/00, G01N 33/38

(54) **BETONELEMENT**

(71) Anmelder: Florack Bauunternehmung GmbH, 52525 Heinsberg (DE)
(72) Erfinder: Blödow, David, 52525 Heinsberg (DE); Martilotti, Dominik-Sergio, 52525 Heinsberg (DE)
(74) Vertreter: dompatent

(57) **Zusammenfassung**

Die Erfindung betrifft ein Betonelement (10) mit einem Speicherelement (12), wobei das Speicherelement (12) Daten enthält, die die technische Beschaffenheit des Betonelements (10) betreffen und die die Eignung des Betonelements (10) für eine Wiederverwendung nach einer Demontage im Rahmen eines Recyclings in einem neuen Bauprojekt beschreiben, wobei das Speicherelement (12) ferner Daten enthält, die für eine zerstörungsfreie Demontage des Betonelements (10) notwendig sind. Die Erfindung betrifft ferner ein Recycling-Planungssystem (16) für Betonelemente (10) sowie ein Verfahren zur Demontage eines Bauwerks, das Betonelemente (10) aufweist.

## Beschreibung

Die Erfindung betrifft ein Betonelement, ein Recycling-Planungssystem für Betonelemente sowie ein Verfahren zur Demontage eines Bauwerks, das Betonelemente aufweist.

Es ist bekannt, Bauwerke aus Betonelementen, beispielsweise aus Betonfertigteilen, zu bauen. Betonfertigteile können beispielsweise in einem Werk vorgefertigt werden und sodann zu einer Baustelle transportiert und in einem Bauwerk verbaut werden. Betonfertigteile können beispielsweise verwendet werden, um Wände, Deckenplatten, Treppen oder Stützen herzustellen.

Problematisch ist, dass die Herstellung von Beton umweltschädlich ist. Ein wesentlicher Bestandteil von Beton ist Zement. Bei der Herstellung von Zement wird Kalkstein bei sehr hohen Temperaturen gebrannt, wobei sehr viel Energie verbraucht wird. Auch wird beim Brennen direkt CO2 freigesetzt, auch bei der chemischen Reaktion selbst und nicht nur lediglich durch den Energieverbrauch. Insgesamt verursacht die Zementproduktion etwa 7 bis 8 Prozent der weltweiten CO2-Emissionen. Hinzu kommt, dass für Beton oft auch große Mengen Sand und Kies gebraucht werden, was zusätzlich die Umwelt belastet.

Dennoch wird zukünftig Beton als Baustoff aufgrund seiner konstruktiven Eigenschaften notwendig sein.

Es gibt zahlreiche Ansätze, die Verwendung von Beton umweltfreundlicher zu gestalten. Unter anderem ist es auch bekannt, Abbruchmaterial von bestehenden Gebäuden zu recyceln.

Problematisch hierbei ist, dass zu Betonelementen, die in einem bestehenden Bauwerk demontiert werden, wenig bis keine Informationen zur Verfügung stehen. Derartige Informationen über ein Betonelement sind jedoch für seine erneute Verwendung in einem neuen Bauwerk sehr wichtig. Beispielsweise ist es wichtig zu wissen, wie viel Stahlbewehrung ein Betonelement aufweist, um seine Tragfähigkeit beurteilen zu können. Weiterhin kann ein Betonelement im Laufe seiner Verwendung in einem Bauwerk schädigenden Umwelteinflüssen ausgesetzt worden sein (beispielsweise Feuchtigkeit). Dies kann ebenfalls seine Eignung für eine weitere Verwendung in einem neuen Bauwerk beeinflussen.

Aufgabe der Erfindung ist es, ein Betonelement bereitzustellen, das eine verbesserte Recyclingfähigkeit aufweist. Auch soll ein Recycling-Planungssystem für Betonelemente sowie ein Verfahren zur Demontage eines Bauwerks, das Betonelemente aufweist, bereitgestellt werden.

Die Lösung der Aufgabe folgt durch die Merkmale der Ansprüche 1, 5 und 6.

Das erfindungsgemäße Betonelement weist ein Speicherelement auf, das Daten enthält, die die technische Beschaffenheit des Betonelements betreffen und die die Eignung des Betonelements für eine Wiederverwendung nach einer Demontage im Rahmen eines Recyclings in einem neuen Bauprojekt beschreiben.

Bei dem Speicherelement kann es sich beispielsweise um einen Speicherchip handeln, dessen Daten über eine Datenverbindung auslesbar sind. Hierbei kann es sich beispielsweise um eine RFID-Verbindung, insbesondere eine passive RFID-Kommunikation handeln. Das Speicherelement muss somit keine Batterie aufweisen und ist somit bevorzugt sehr lange haltbar und funktionsfähig.

Die in dem Speicherelement enthaltenen Daten können beispielsweise die Menge und Art der Stahlbewehrung betreffen, die das Betonelement aufweist. Hierdurch stehen Informationen über die Tragfähigkeit des Betonelements zur Verfügung, die für seine Wiederverwendung im Rahmen eines Recyclings verwendet werden können. Das Speicherelement kann weiterhin eine oder mehrere der folgenden Informationen enthalten:
- Materialinformationen zur Verwendung des Betons, wie die Betonart (bspw. Normalbeton, Stahlbeton, Recyclingbeton, Sichtbeton)
- Festigkeitsklasse des verwendeten Betons (bspw. C25/25 - C30/37: Wohnhäuser, C35/45 - C50/60: Brücken, Parkhäuser etc.)
- Genaue geometrische Abmessungen des individuellen Bauteils
- Informationen zu verbauten Versorgungsleitungen im Inneren der Betonelemente
- Verwendete Montageteile sowie Montage- und Demontage-Anweisungen

Das Speicherelement enthält ferner Daten, die für eine zerstörungsfreie Demontage des Betonelements notwendig sind. Hierbei kann es sich beispielsweise um Informationen darüber handeln, an welchen Stellen das Betonelement vom bestehenden Bauwerk zu trennen ist, damit es zerstörungsfrei demontiert werden kann. Weiterhin können Informationen darüber enthalten sein, an welchen Stellen das Betonelement angehoben werden muss, um zerstörungsfrei demontiert und abtransportiert zu werden. Das Speicherelement kann weiterhin eine oder mehrere der folgenden Informationen für eine zerstörungsfreie Demontage enthalten:
Dokumentation der Verbindungen und Montageart, wie z.B.:
   - Verbindungsart der Bauteile (geschraubt, gesteckt, geklemmt, verklebt etc.)
   - Zugänglichkeit der Verbindung - z.B. Wo befinden sich Schrauben, Haken, Klammern? Sind diese sichtbar oder verdeckt?
   - Reihenfolge der Montage (In welche Reihenfolge wurden die Elemente montiert) sowie Reihenfolge der Demontage (entsprechend der Montage müssen die Elemente demontiert werden)
   - Erforderliche Werkzeuge und Hilfsmittel für die Demontage
Bauteilkatalog/Bauteilpass:
   - Eindeutige Kennzeichnung oder Nummerierung der Elemente
   - Alterungszustand und ggf. Belastungshistorie (bei tragenden Teilen) Gebäudestruktur und Bauweise
   - Tragwerksplanung: Welche Teile tragen die Last? Was kann zuerst abgebaut werden, ohne Stabilität zu gefährden?
   - Modularisierung: Sind Bauteile als Module zusammengefasst?
   - 3D Modell und Konstruktionspläne: Wie greifen die Teile ineinander?
Außerdem:
   Materialzusammensetzung, Wiederverwendbarkeit
   Das Speicherelement im erfindungsgemäßen Betonelement ermöglicht es somit, vor der Demontage des Betonelements Informationen über seine technische Beschaffenheit zu erhalten und seine Eignung für eine Wiederverwendung im Rahmen eines Recyclings in einem neuen Bauprojekt zu beurteilen. Dies kann auch nach einem sehr langen Zeitraum, beispielsweise nach vielen Jahrzehnten, erfolgen.

Alternativ oder zusätzlich zu einem Speicherelement im Betonelement, können Informationen zur technischen Beschaffenheit des Betonelements, die seine Eignung für eine Wiederverwendung im Rahmen eines Recyclings betreffen, auch an einem anderen Ort, beispielsweise in einem Cloud-Speicher hinterlegt sein. Insbesondere können diese Informationen mit einem 3D-Modell des Bauwerks verknüpft sein. Hierdurch ist es möglich, zukünftig zu wissen, wo welches Betonelement verbaut ist. Bevorzugt erfolgt eine eindeutige Zuordnung des Betonelements zum 3D-Modell, sodass eine digitale Karte aller Betonelemente erstellt wird.

Die im Speicherelement und/oder dem externen Datenspeicher, beispielsweise der Cloud, enthaltenen Daten für eine zerstörungsfreie Demontage des Beton-elements sind zusätzlich hilfreich, da sie den Rückbau und die Wiederverwendung der Betonelemente erheblich vereinfachen.

Es bevorzugt, dass das Betonelement einen Sensor zur Erfassung von Eigenschaften des Betonelements aufweist. Hierbei kann es sich beispielsweise um einen Feuchtigkeitssensor handeln.

Besonders bevorzugt ist es, dass durch den Feuchtigkeitssensor eine Erfassung der Feuchtigkeit des Betonelements, insbesondere über einen längeren Zeitraum, erfolgt. Bei dem längeren Zeitraum kann es sich beispielsweise um die gesamte Lebenszeit des Betonelements handeln, die mit dem Zeitpunkt seiner Herstellung beginnt und mit seiner Demontage oder seiner späteren Vernichtung endet. Es ist somit der zeitliche Verlauf der Feuchtigkeit im Betonelement, während seiner gesamten Historie bekannt. Sollte das Betonelement beispielsweise in der Vergangenheit über einen längeren Zeitraum einer erhöhten Feuchtigkeit ausgesetzt gewesen sein, kann dies seine Eignung für eine Wiederverwendung im Rahmen eines Recyclings negativ beeinflussen. Sofern hier erlaubte Schwellwerte überschritten werden, könnte beispielsweise eine Wiederverwendung dieses Betonelements ausgeschlossen werden. Die erfassten Sensordaten werden bevorzugt im Speicherelement gespeichert.

Das Speicherelement im Betonelement und/oder der externe Datensprecher kann auch Daten enthalten, die CO2-Emissionswerte betreffen. Es kann somit festgehalten werden, wieviel CO2 bei der Herstellung eines Betonelements entstanden ist. Diese Daten können zur Bewertung der ökologischen Nachhaltigkeit eines Betonelements oder eines gesamten Bauwerks genutzt werden.

Die Erfindung betrifft ferner ein Recycling-Planungssystem für Betonelemente. Hierbei kann es sich um eine Vielzahl von Betonelementen handeln, wie sie bisher beschrieben wurden. Ferner umfasst das Recycling-Planungssystem einen Datensprecher, der datentechnisch mit den Betonelementen verbindbar ist. Hierbei kann es sich beispielsweise um einen Datenspeicher in einer Cloud handeln. Auf dem Datenspeicher ist ein Modell eines Bauwerks gespeichert, in dem die Betonelemente verbaut sind. Dieser Datenspeicher kann zusätzlich oder alternativ zum Speicherelement in dem Betonelement selbst technische Informationen über das Betonelement und Informationen über seine zerstörungsfreie Demontage enthalten. Weitere Details zur Verwendung von Informationen über Betonelemente, die in einem solchen externen Datensprecher gespeichert sind, wurden bereits in der vorliegenden Anmeldung ausführlicher beschrieben.

Die Erfindung betrifft ferner ein Verfahren zur Demontage eines Bauwerks, das Betonelemente aufweist, mit den folgenden Schritten:
Demontieren mindestens eines Betonelements, sodass dieses vom Bauwerk getrennt werden kann,
wobei das Betonelement mindestens ein Speicherelement aufweist, das technische Informationen über das Betonelement enthält,
Auslesen der technischen Informationen dieses Speicherelements vor, nach und/oder während der Demontage des Betonelements,
Zuführen des demontierten Betonelements zu einer weiteren Verwendung in einem anderen Bauwerk auf Basis der ausgelesenen Daten über das Betonelement.

Beim Demontieren des Betonelements können beispielsweise Informationen aus dem Speicherelement, die seine zerstörungsfreie Demontage betreffen, berücksichtigt werden.

Das erfindungsgemäße Verfahren ermöglicht die gleichen Vorteile wie das erfindungsgemäße Betonelement und erleichtert die Wiederverwendung eines Betonelements im Rahmen eines Recyclings.

Im Rahmen des erfindungsgemäßen Verfahrens können ferner Informationen aus dem Speicherelement an eine Datenbank, beispielsweise einen Cloudspeicher, die ein 3D-Modell des Bauwerks enthält, übermittelt werden. Diese Informationen können dem entsprechenden Betonelement im 3D-Modell zugeordnet werden. Das Demontieren des Beton-Elements sowie seine Zuführung zu einer weiteren Verwendung kann sodann unter Berücksichtigung der Daten aus dem 3D-Modell erfolgen.

Im Folgenden wird eine bevorzugte Ausführungsform der Erfindung anhand einer Figur erläutert.

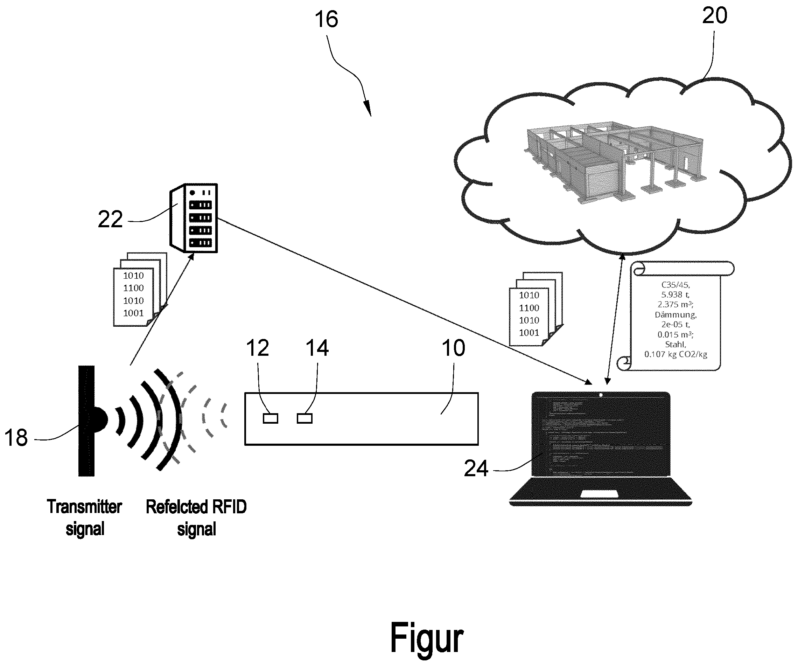

Die Figur zeigt eine Ausführungsform eines erfindungsgemäßen Recycling-Planungssystems (16). Dieses weist eine Vielzahl von Betonelementen (10) auf, von denen in der Figur 1 lediglich eins schematisch dargestellt ist. Dieses weist ein Speicherelement (12) sowie einen Sensor (14) auf. Das Speicherelement (12) enthält Daten, die die technische Beschaffenheit des Betonelements (10) betreffen und die die Eignung des Betonelements (10) für eine Wiederverwendung nach einer Demontage im Rahmen eines Recyclings in einem neuen Bauprojekt beschreiben. Das Speicherelement (12) enthält ferner Daten, die für eine zerstörungsfreie Demontage des Betonelements (10) notwendig sind.

Der Sensor (14) kann beispielsweise ein Feuchtigkeitssensor sein.

Sowohl die Daten des Speicherelements (12) als auch des Sensors (14) können über eine passive RFID-Verbindung ausgelesen werden. Hierzu sendet ein Transmitter ein magnetisches oder elektromagnetisches Signal aus, durch das die Daten in bekannter Weise ausgelesen werden können. Dies erfolgt vorzugsweise, ohne dass das Speicherelement (12) und der Sensor (14) eine Batterie aufweisen müssen.

Die ausgelesenen Daten können an einen Cloud-Datenspeicher (20) übermittelt werden, der ein dreidimensionales Modell des Bauwerks enthält, in dem das Betonelement (10) verbaut ist.

Bei allen Ausführungsformen der vorliegenden Erfindung können alternativ oder zusätzlich zum Speicherelement (12) im Betonelement (10) selbst sämtliche Daten, die die Wiederverwendbarkeit des Betonelements (10) und/oder seine zerstörungsfreie Demontage und/oder die Daten des Sensors (14) auf einem weiteren externen Rechner (22) bzw. einer dort vorhandenen Speichervorrichtung gespeichert sein. Hierdurch ist es möglich, dass ein Nutzer über seinen Zugangsrechner (24) auf diese Daten auf dem Rechner (22) zugreifen kann. Alternativ oder zusätzlich kann ein Zugriff auf die in der Cloud (20) befindlichen Daten erfolgen.

Ferner können alternativ oder zusätzlich die genannten Daten auch im Rahmen einer Block Chain zentral gespeichert werden. Dies bietet den Vorteil, dass eine Speicherung über viele Jahrzehnte auf einfache Weise möglich ist, da sie nicht von einer einzigen Instanz abhängt, auf der die Daten gespeichert sind.

Die Daten aus dem Speicherelement (12) und dem Sensor (14) können sodann im Rahmen eines Recyclings bei einer Wiederverwendung des Betonelements (10) in einem anderen Bauwerk berücksichtigt werden. Dies gilt ebenso für die Daten aus dem dreidimensionalen Modell des Bauwerks, die in dem Cloud-Datenspeicher (20) gespeichert sind.

## Patentansprüche

1. Betonelement (10),
mit einem Speicherelement (12),
wobei das Speicherelement (12) Daten enthält, die die technische Beschaffenheit des Betonelements (10) betreffen und die die Eignung des Betonelements (10) für eine Wiederverwendung nach einer Demontage im Rahmen eines Recyclings in einem neuen Bauprojekt beschreiben,
wobei das Speicherelement (12) ferner Daten enthält, die für eine zerstörungsfreie Demontage des Betonelements (10) notwendig sind.

2. Betonelement (10) nach Anspruch 1, **dadurch gekennzeichnet, dass** das Betonelement (10) einen Sensor zur Erfassung von Eigenschaften des Betonelements (10) aufweist.

3. Betonelement (10) nach Anspruch 2, **dadurch gekennzeichnet, dass** der Sensor (14) ein Feuchtigkeitssensor ist.

4. Betonelement (10) nach Anspruch 3, **dadurch gekennzeichnet, dass** durch den Feuchtigkeitssensor (14) eine Erfassung der Feuchtigkeit des Betonelements (10) insbesondere über einen längeren Zeitraum erfolgt.

5. Recycling-Planungssystem (16) für Betonelemente (10) mit einer Vielzahl von Betonelementen (10) nach einem der Ansprüche 1 - 4, einem Datenspeicher, der Datentechnisch mit den Betonelementen (10) verbindbar ist, wobei auf dem Datenspeicher ein Model eines Bauwerks gespeichert ist, in dem die Betonelemente (10) verbaut sind.

6. Verfahren zur Demontage eines Bauwerks, das Betonelemente (10) aufweist, mit den Schritten:
Demontieren mindestens eines Betonelements (10), sodass dieses vom Bauwerk getrennt werden kann,
wobei das Betonelement (10) mindestens ein Speicherelement (12) aufweist, das technische Informationen über das Betonelement (10) enthält,
Auslesen der technischen Informationen dieses Speicherelements (12) vor, nach und/oder während der Demontage des Betonelements (10),
Zuführen des demontierten Betonelements (10) zu einer weiteren Verwendung in einem anderen Bauwerk auf Basis der ausgelesenen Daten über das Betonelement (10).

7. Verfahren zur Demontage eines Bauwerks, nach Anspruch 6, **gekennzeichnet durch** die weiteren Verfahrensschritte:
Übermitteln der Informationen aus dem Speicherelements (12) über das Betonelement (10) an eine Datenbank, die ein 3-D-Model des Bauwerks enthält,
Zuordnen der Informationen zum entsprechenden Betonelement (10) im 3-D-Model, wobei das Demontieren des Betonelements (10) und sein Zuführen zu einer weiteren Verwendung unter Berücksichtigung der Daten aus dem 3-D-Model erfolgt.
